# EUROPEAN PATENT SPECIFICATION

(11) **EP 0 361 493 B1**
(45) Date of publication and mention of the grant of the patent: **23.11.1994**
(21) Application number: 89118025.9
(22) Date of filing: 29.09.1989
(51) Int. Cl.: C07C 43/10

(54) **Diethers usable in the preparation of Ziegler-Natta catalysts and their preparation**
Diether, verwendbar bei der Herstellung von Ziegler-Natta-Katalysatoren, und ihre Herstellung
Diéthers utilisables dans la préparation des catalyseurs Ziegler-Natta et leur préparation

(30) Priority: 30.09.1988 IT 2215288
(43) Date of publication of application: 04.04.1990
(73) Proprietor: Himont Incorporated, New Castle County Delaware (US)
(72) Inventor: Agnes, Giovanni, I-28100 Novara (IT); Borsotti, Giampietro, I-28100 Novara (IT); Schimperna, Giuliana, I-20162 Milano (IT); Barbassa, Elisabetta, I-27058 Voghera Pavia (IT)
(74) Representative: Zumstein, Fritz, Dr.

(56) References cited:
- DE-C- 888 999
- US-A- 3 290 387
- CHEMICAL ABSTRACTS, vol. 99, no. 5, Columbus, Ohio, USA WHALON, MICHAEL R. et al. "A remarkable consistency in conformational preference for a series of 1,3-di- substituted-2,2-dimethylpro- panes" page 478, column 1, Abstract- -no. 37 911t
- CHEMICAL ABSTRACTS, vol. 84, no. 24, Columbus, Ohio, USA MC ALEES, ALAN J. et al. "Complexes of titanium tetra- chloride with terdentate triped ligands. Competition among oxygen, sulfur and nitrogen for coordination sites on titanium" page 593, column 1, Abstract- -no. 173 108d

## Description

The present invention refers to a new class of diethers.

The diethers of the invention are useful as additives for fuels (where they produce an increase in the octane number), as solvents, as a complexing agent for metal ions, and in the preparation of Ziegler-Natta catalysts.

Heretofore only unsubstituted diethers were considered as useful compounds as solvent. For example DE-C-888 999 discloses the preparation and the use of 1,3-dimethoxypropane as solvent having a boiling-point of 105°C, useful for polar compounds.

Chemical Abstracts, vol. 99 (1983), abstract No. 37 911t discloses the compound 2,2-dimethyl-1,3-dimethoxypropane and cites its preferred conformation by means of a H¹-NMR study.

US-A-4,374,282 describes alkyl ethers having formula: 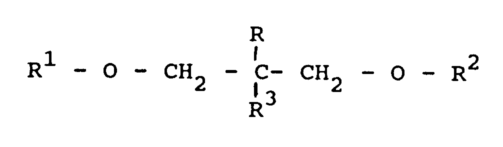
wherein R, R¹, R² and R³ are independently alkyl radicals containing from 1 to about 22, preferably 6 to 16, carbon atoms; the alkyl radicals are straight or branched.

Said prior art documents do not teach the use of diethers as electron-donors in catalyst components for the Ziegler-Natta catalysts.

The diethers of the present invention have the general formula: 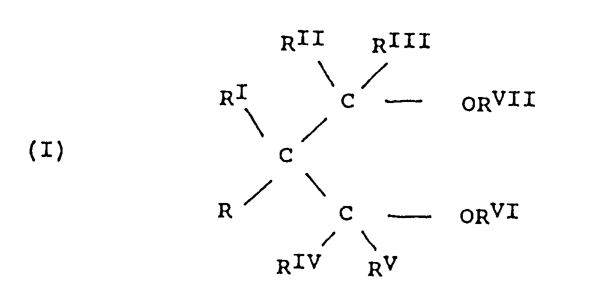
where R, R^{I}, R^{II}, R^{III}, R^{IV} and R^{V}, same or different, are H or linear or branched alkyl, cycloalkyl, aryl, alkylaryl or arylalkyl radicals with 1-18 carbon atoms, provided that R and R^{I} are not both H and when R and R^{I} are alkyl radicals, they have 3 or more carbon atoms; R^{VI} and R^{VII}, the same or different, are linear or branched alkyl, cycloalkyl, aryl, alkylaryl radicals with 1 - 18 carbon atoms; two or more of R to R^{VII} can be bonded to form a cyclic structure; with the proviso that there are excluded diethers in which all of R^{II}, R^{III}, R^{IV} and R^{V} are H; and all of R, R^{I}, R^{VI} and R^{VII} are alkyl radicals as defined above.

Preferably R^{VI} and R^{VII} are alkyl radicals with 1 - 6 carbon atoms, R^{II}, R^{III}, R^{IV} and R^{V} are hydrogen and R and R^{I} are aryl, alkylaryl or arylalkyl radicals as defined above.

The new diethers may be prepared according to various methods. For example, they may be prepared according to known etherification reactions such as the ones listed below, starting from the corresponding diols of general formula (II) 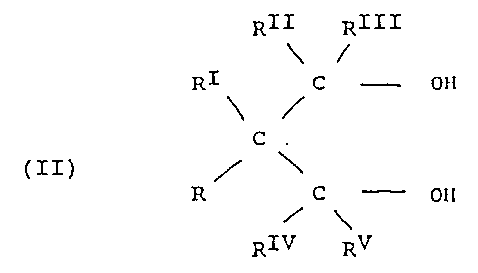
1) Reaction of diols of formula II or the corresponding alkaline alcoholates with compounds of formula R^{VI}-X, R^{VII}-X or their mixtures (where X = Cl, Br, I, C₆H₅-SO₃, p-CH₃C₆H₄SO₃, CH₃SO₃), wherein R, R^{I}, R^{II}, R^{III}, R^{IV} and R^{V} have the same meaning as set forth above.
2) Reaction of diols of formula II with dialkyl sulfates of formula R₂^{VI} SO₄ or R₂^{VII} SO₄ in alkaline environment.
3) Reaction of derivatives of general formula III, using known techniques, starting from the diols of formula II 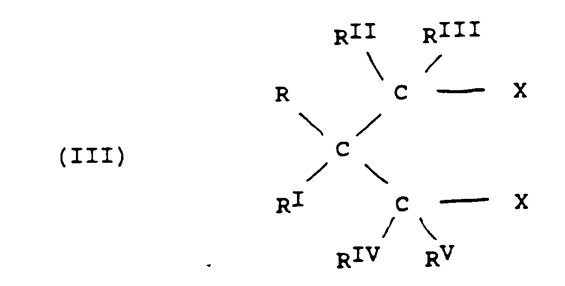 (wherein R to R^{V} and X have the meaning as indicated above) with R^{VI}-OM and R^{VII} OM alcoholates, wherein M = Na, K, Mg, Ca or mixtures thereof.
4) Thermal or catalytic dehydration of mixtures of diols of general formula (II) with R^{VI} OH or R^{VII} OH alcohols or mixtures thereof.
   These and other suitable methodologies are described in:
   1) Houben Weil - Metoden der Organischen Chemie Vol VI/₃ Verlag ed. Stuttgard 1965.
   2) G.W.Gokel and Coll. Syntesis 1976, 168.
   3) G. Johns and Coll. ibid. 1976, 515.
   4) D. Achet and Coll. ibid. 1986, 642.

   The diols of general formula (II) may in turn be synthetized, for example, according to known methods such as the reduction of the corresponding diesters, dialdehydes, diketones, ketoaldehydes or dicarboxylic acids, having the general formula IV and V, and ketoesters and aldehyde esters of general formula (VI) 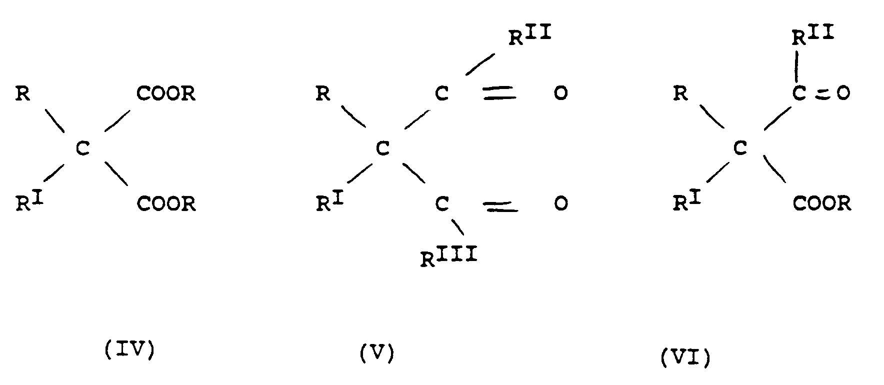
   (where the radicals R, R^{I}, R^{II}, R^{III} have the meaning as indicated above).

Examples of these methods are described in:
- H. Adkins, Organic Reactions 8, 1 (1954)
- N.G. Gaylord, Reduction with Complex Metal Hydrides, Interscience Publishers, N.Y., London 1956.
- R. F. Nystrom, W. G. Brown, J. Am. Chem. Soc. 69, 1197 (1947).

Furthermore diols of formula II (wherein R^{II}, R^{III}, R^{IV}, and R^{V} are H) may also be prepared from aldehydes of general formula VII 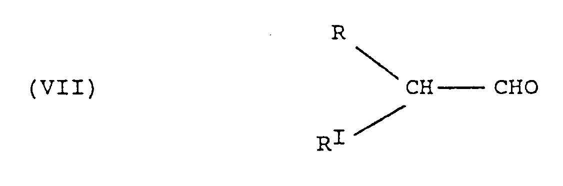
wherein R and R^{I} have the same meaning as already specified, by action of alkaline formaldehyde according to the Cannizzaro reaction (see for example Organic Reactions Vol II, pag. 94 -J. While ed. - N.Y., 1944).

Diols of general formula II may easily be converted into the corresponding III derivates by known methods (see Houben Weil, Methoden der organischen Chemie, Band V/3, V/4, IX; Verlag ed. Stuttgard).

The following examples illustrate the following ethers of the invention and methods of preparing same:
- 2,2-diphenyl-1,3-dimethoxypropane
- 2,2-dibenzyl-1,3-dimethoxypropane
- 2,2-bis(cyclohexylmethyl)1,3-dimethoxypropane
- 1,1-bismethoxymethylcyclohexane
- (+/-) 2,2-bis(methoxymethyl)norbornane (racemic mixture).

Other examples of ethers are:
- 2-isopropyl-2-cyclohexylmethyl-1,3-dimethoxypropane
- 2-isopropyl-2-cyclohexyl-1,3-dimethoxypropane
- 2-isopropyl-2-cyclopentyl-1,3-dimethoxypropane
- 2,2-dicyclopentyl-1,3-dimethoxypropane
- 2,2-dicyclohexyl-1,3-dimethoxypropane

### Reference Example

### Preparation of 2,2-diisobutyl-1,3-dimethoxypropane.

a) Preparation of diisobutyl ethyl malonate.
   Into a 250 ml flask, equipped with an agitator, refrigerant, charge funnel, thermometer and tube for the introduction of gases, were charged, under nitrogen flow, 100 g anhydrous ethanol and 5 g (0.22 moles) Na.
   When the dissolution of Na was complete, 16 g (0.1 moles) diethylmalonate was introduced, and stirred at room temperature for a few minutes. Then 28 g isobutyl bromide (0.21 moles) was added and the mixture was refluxed with agitation for 6 hours. Subsequently, 7.5 g dry sodium ethylate (0.12 moles) and 14 g isobutyl bromide (0.1 moles) were added. The agitation and the reflux heating was continued for 8 more hours.
   Most of the solvent was distilled off at reduced pressure [6665 Pa (50 mm Hg)], and the remainder was extracted with 200 ml hexane. The distilled hexane solution provided 15.5 g of diisobutyl ethylmalonate with a boiling point of 145-146°C 2666 Pa (20 mm Hg). The product has a gas-chromatographic purity (main peak area) of 97.5%, and coincides with a sample of diisobutyl ethylmalonate prepared according to Bently and Perkin, J. Chem. Soc. 73, 61.
b) Preparation of 2,2-diisobutyl-1,3-propandiol.
   Into the same apparatus as described above in a) were introduced, under nitrogen flow, 100 ml diethyl ether and 3 g LiAlH₄ (0.079 moles).
   Then dropwise over a period of one hour while maintaining vigorous agitation, 15.5 g diisobutyl ethyl malonate from a) above was added and the mixture refluxed for 30 minutes.
   The reaction mixture was then poured into a vessel containing 100 g ice acidified with dil. HCl and extracted with 3 portions of 100 ml of ethyl ether.
   The ether was evaporated and 10 g of a raw material was produced which, when crystallized from hexane, gave 8.5 g of 2,2-diisobutyl-1,3-propandiol with melting point 75-77°C and an elemental analysis of C=70.3% and H=12.6%. The theoretical value for C₁₁H₂₄O₂ is C=70.21% H=12.7%.
c) Preparation of 2,2-diisobutyl-1,3-dimethoxypropane.
   Into the same apparatus as described above in a) was introduced, under nitrogen, 8.5 g (0.06 moles) 2,2-diisobutyl-1,3-propandiol, 200 ml dioxane and 15.4 g (0.136 moles) potassium tert-butylate.
   The mixture was stirred at room temperature for 30 minutes and then 20 g CH₃I (0.14 moles) was added dropwise. During this procedure, the temeperature rises spontaneously to 50°C.
   After 2 hours, an additional quantity of potassium tert-butylate (154 g, 0.136 moles) and of CH₃I (20 g, 0.14 moles) was added, and the mixture refluxed for 1 hour.
   The reaction mass was filtered and the filtrate distilled at reduced pressure. Among other products, 7.4 g of 2,2-diisobutyl-1,3-dimethoxypropane having a boiling point 100-101°C 2933 Pa (22 mm-Hg) which by gas layer cromatography (GLC) shows a purity of 99% (chromatographic peaks area) was obtained.
   n^{D} ₂₀ = 1.4337.
   ¹HNMR (300 MH_{z}, CDCl₃, TMS as internal standard):
   signals at
   0.89 ppm, doublet 12 H
   1.21 ppm, doublet 4 H
   1.68 ppm, multiplet 2 H
   3.16 ppm, singlet 4 H
   3.26 ppm. singlet 6 H

### Example 1

Using the procedures of a), b) and c) of the reference example 2,2-dibenzyl-1,3-dimethoxypropane p.f. 105°C (from petroleum ether) was obtained.

### Example 2

### Preparation of 2,2-bis(cyclohexymethyl)-1,3-dimethoxypropane.

Into a stainless steel autoclave provided with an anchor agitation system, 5.8 g (0.02 moles) of (C₆H₅CH₂)₂C(CH₂OCH₃)₂ prepared according to reference example, 100ml n-hexane and 10 g Raney Ni washed by decanting with 3 parts 50 cc anhydrous ethanol and subsequently with 3 parts 50 cc of hexane, were introduced.

The autoclave was pressurized with 17.10⁵ Pa (17 atm.) of hydrogen and was heated to 135°C (internal temperature) for 8 hours with agitation.

After cooling, the reaction mixture, was filtered from the catalyst and vacuum evaporated, to yield 5.9 g of a colorless oil with a purity of 99% n^{D}₂₀ = 1.4790. The only compound detectable by thin layer chromatography (TLC), was 2,2-bis(cyclohexyl,methyl)-1,3-dimethoxypropane.
¹HNMR(300 MHz, CDCl₃, TMS as internal standard).
Signals at:
0.96 ppm multiplet 4 H
1.18 ppm multiplet 12 H
1.63 ppm multiplet 10 H
3.15 ppm singlet 4 H
3.27 ppm singlet 6 H

### Example 3

### Preparation of 2,2-diphenyl-1,3-dimethoxypropane.

a) Preparation of 2,2-diphenyl-1,3-propandiol.
   Into the same apparatus described in reference example (a)), 10.6 g (0.054 moles) of (C₆H₅)₂CHCHO (Fluka), 4.03 g (0.028 moles) K₂CO₃, 10 cc water, 13.2 ml acqueous formaldehyde at 40% (0.176 moles) and 35 ml ethanol at 99% purity were introduced.
   The mixture was stirred and refluxed for 6 hours, cooled and diluted with 200 ml of water. The precipitate thus formed was filtered, washed with water and crystallized from benzene to give 9.6 g of 2,2-diphenyl-1,3-propandiol with a m.p. 102-104°C.
b) Preparation of 2,2-diphenyl-1,3-dimethoxypropane.
   Into the same apparatus as described in a) was charged 9.6 g 2,2-diphenyl-1,3-propandiol dissolved in 400 ml anhydrous tetrahydrofuran and stirred under nitrogen with 3.8 g NaH (55% NaH dispersed in vaseline oil) until the production of hydrogen stops. Over a period of 20 minutes, 9.6 ml CH₃I was added and stirring continuously for 2 hours. Most of THF was distilled off; then the product is diluted with water (200 ml) and extracted with two 50 ml portions of diethyl ether. The ether extract gives, by vacuum distillation, 3.5 g of 2,2-diphenyl-1,3-dimethoxypropane having boiling point of 188°C-190°C 2666 Pa (20 mm Hg) which was unitary by TLC-chromatography and having
   n^{D}₂₀ = 1.5558.

According to the same procedure described above in a) and b) the following compounds were prepared starting respectively from hexahydrobenzenaldehyde and norbornan-2-carboxhaldehyde.
A) 1,1-dismethoxymethylcyclohexane
   boiling point 97°-98°C 2666 Pa (20 mm Hg); n^{D}₂₀ = 1.4487
   ¹HNMR (300 MHz, CDCl₃, TMS as internal standard):
   signals at:
   1.36 ppm multiplet 10 H
   3.20 ppm singlet 4 H
   3.29 ppm singlet 6 H
B) (+/-) 2,2-dismethoxymethylnorbornane
   boiling point 106°-108°C 2666 Pa (20 mm Hg); n^{D}₂₀ = 1.4659
   ¹HNMR (300 MHz, CDCl₃, TMS as internal standard)
   Signals at:
   0.72 ppm doublet 1 H
   1.14 ppm doublet 1 H
   1.06 ppm multiplet 1 H
   1.34 ppm multiplet 2 H
   1.51 ppm multiplet 3 H
   1.97 ppm singlet (broad) 1 H
   2.15 ppm singlet (broad) 1 H
   3.06 ppm system AB 1 H
   3.14 ppm system AB 1 H
   3.33 ppm system AB 1 H
   3.36 ppm system AB 1 H
   3.29 ppm multiplet 6 H

## Claims (Claims for the following Contracting State(s): BE, DE, FR, GB, IT, NL)

1. Diethers of formula I 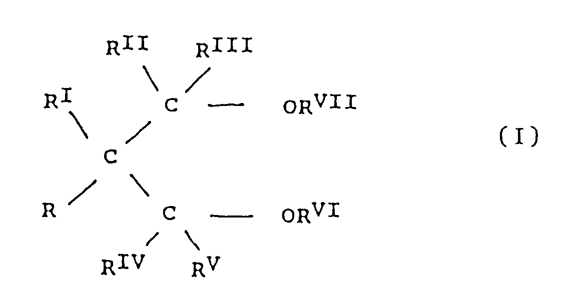 where R and R^{I}, R^{II}, R^{III}, R^{IV} and R^{V} same or different, are H or linear or branched alkyl, cycloalkyl, aryl, alkylaryl or arylalkyl radicals with 1 - 18 carbon atoms, provided that R and R^{I} are not both H and when R and R^{I} are alkyl radicals, they have three or more carbon atoms;
R^{VI} and R^{VII} are the same or different and are linear or branched alkyl, cycloalkyl, aryl, alkylaryl radicals with 1 - 18 carbon atoms; two or more of R to R^{VII} can be bonded to form a cyclic structure; with the proviso that there are excluded diethers in which all of R^{II}, R^{III}, R^{IV} and R^{V} are H; and all of R, R^{I}, R^{VI} and R^{VII} are alkyl radicals as defined above.

2. Diethers according to claim 1 wherein R^{VI} and R^{VII} are alkyl radicals with 1 - 6 carbon atoms, R^{II}, R^{III}, R^{IV} and R^{V} are hydrogen and R and R^{I} are aryl, alkylaryl or arylalkyl radicals according to claim 1.

3. Diethers according to claim 2 selected from the group consisting of:
- 2,2-diphenyl-1,3-dimethoxypropane
- 2,2-dibenzyl-1,3-dimethoxypropane
- 2,2-bis(cyclohexylmethyl)-1,3-dimethoxypropane
- 2-isopropyl-2-cyclohexylmethyl-1,3-dimethoxypropane
- 2-isopropyl-2-cyclohexyl-1,3-dimethoxypropane
- 2-isopropyl-2-cyclopentyl-1,3-dimethoxypropane
- 2,2-dicyclopentyl-1,3-dimethoxypropane
- 2,2-dicyclohexyl-1,3-dimethoxypropane

4. A process for preparing a diether of formula I as defined in claim 1 comprising the step of reacting:
a diol of formula II 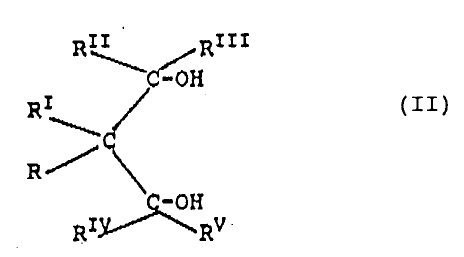 with compounds of formula R^{VI}-X, R^{VII}-X or their mixtures (where X = Cl Br, I, C₆H₅-SO₃, p-CH₃C₆H₄-SO₃, CH₃SO₃), wherein R and R^{I}, R^{II}, R^{III}, R^{IV} and R^{V} have the same meaning as set forth in claim 1.

5. The process according to claim 4 wherein R^{VI} and R^{VII} are alkyl radicals with 1 to 6 carbon atoms and R^{II}, R^{III}, R^{IV} and R^{V} are hydrogen and R and R^{I} are aryl, alkylaryl or arylalkyl radicals according to claim 1.

6. The process according to claim 5 for preparing a diether selected from the group consisting of:
- 2,2-diphenyl-1,3-dimethoxypropane
- 2,2-dibenzyl-1,3-dimethoxypropane
- 2,2-bis(cyclohexylmethyl)-1,3-dimethoxypropane
- 2-isopropyl-2-cyclohexylmethyl-1,3-dimethoxypropane
- 2-isopropyl-2-cyclohexyl-1,3-dimethoxypropane
- 2-isopropyl-2-cyclopentyl-1,3-dimethoxypropane
- 2,2-dicyclopentyl-1,3-dimethoxypropane
- 2,2-dicyclohexyl-1,3-dimethoxypropane.

## Claims (Claims for the following Contracting State(s): ES)

1. A process for preparing a diether of formula I 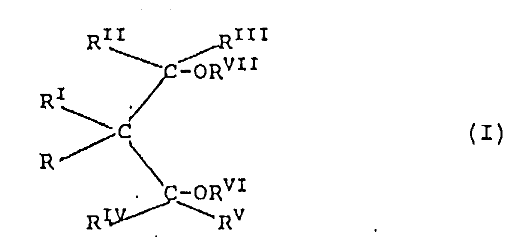 wherein R and R^{I}, R^{II}, R^{III}, R^{IV} and R^{V}, same or different, are H or linear or branched alkyl, cycloalkyl, aryl, alkylaryl or arylalkyl radicals with 1 - 18 carbon atoms; provided that R and R^{I} are not both H and when R and R^{I} are alkyl radicals, they have three or more carbon atoms; R^{VI} and R^{VII,} same or different, are linear or branched alkyl, cycloalkyl, aryl, alkylaryl radicals with 1 - 18 carbon atoms; and two or more of R to R^{VII} can be bonded to form a cyclic structure; with the proviso that there are excluded diethers in which all of R^{II}, R^{III}, R^{IV} and R^{V} are H; and all of R, R^{I}, R^{VI} and R^{VII} are alkyl radicals as defined above; comprising the step of reacting:
a diol of formula II 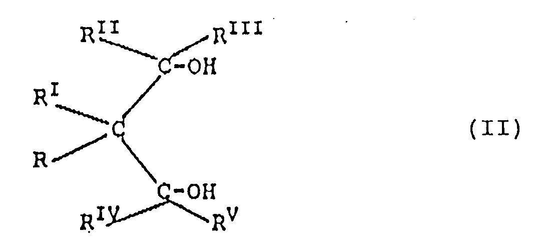 with compounds of formula R^{VI}-X, R^{VII}-X or their mixtures (where X = Cl, Br, I, C₆H₅-SO₃, p-CH₃C₆H₄-SO₃, CH₃SO₃), wherein R and R^{I}, R^{II}, R^{III}, R^{IV} and R^{V} have the same meaning as set forth above.

2. The process according to claim 1 wherein R^{VI} and R^{VII} are alkyl radicals with 1 - 6 carbon atoms and R^{II}, R^{III}, R^{IV} and R^{V} are hydrogen and R and R^{I} are aryl, alkylaryl or arylalkyl radicals according to claim 1.

3. The process according to claim 2 for preparing a diether selected from the group consisting of:
- 2,2-diphenyl-1,3-dimethoxypropane
- 2,2-dibenzyl-1,3-dimethoxypropane
- 2,2-bis(cyclohexylmethyl)-1,3-dimethoxypropane
- 2-isopropyl-2-cyclohexylmethyl-1,3-dimethoxypropane
- 2-isopropyl-2-cyclohexyl-1,3-dimethoxypropane
- 2-isopropyl-2-cyclopentyl-1,3-dimethoxypropane
- 2,2-dicyclopentyl-1,3-dimethoxypropane
- 2,2-dicyclohexyl-1,3-dimethoxypropane

## Patentansprüche (Patentansprüche für folgende(n) Vertragsstaat(en): BE, DE, FR, GB, IT, NL)

1. Diether der Formel I 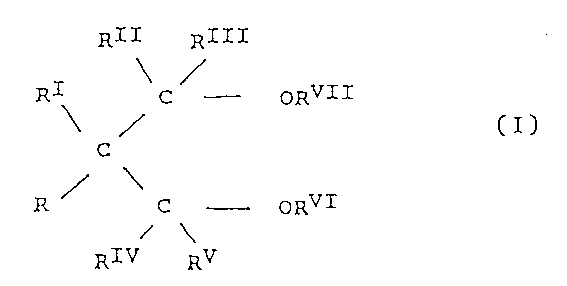 worin R und R^{I}, R^{II}, R^{III}, R^{IV} und R^{V}, gleich oder verschieden, für H oder lineare oder verzweigte Alkyl-, Cycloalkyl-, Aryl-, Alkaryl- oder Aralkylreste mit 1 bis 18 Kohlenstoffatomen stehen, mit der Maßgabe, daß R und R^{I} nicht beide für H stehen, und wenn R und R^{I} Alkylreste sind, diese drei oder mehr Kohlenstoffatome aufweisen; R^{VI} und R^{VII} gleich oder verschieden sind und für lineare oder verzweigte Alkyl-, Cycloalkyl-, Aryl-, Alkarylreste mit 1 bis 18 Kohlenstoffatomen stehen; zwei oder mehrere von R bis R^{VII} unter Bildung einer cyclischen Struktur gebunden sein können; mit der Maßgabe, daß Diether ausgeschlossen sind, worin sämtliche von R^{II}, R^{III}, R^{IV} und R^{V} für H stehen; und sämtliche von R, R^{I}, R^{VI} und R^{VII} Alkylreste, wie vorstehend definiert, sind.

2. Diether gemäß Anspruch 1, worin R^{VI} und R^{VII} Alkylreste mit 1 bis 6 Kohlenstoffatomen bedeuten, R^{II}, R^{III}, R^{IV} und R^{V} Wasserstoff sind und R und R^{I} für Aryl-, Alkaryl- oder Aralkylreste gemäß Anspruch 1 stehen.

3. Diether gemäß Anspruch 2, ausgewählt unter
2,2-Diphenyl-1,3-dimethoxypropan
2,2-Dibenzyl-1,3-dimethoxypropan
2,2-Bis-(cyclohexylmethyl)-1,3-dimethoxypropan
2-Isopropyl-2-cyclohexylmethyl-1,3-dimethoxypropan
2-Isopropyl-2-cyclohexyl-1,3-dimethoxypropan
2-Isopropyl-2-cyclopentyl-1,3-dimethoxypropan
2,2-Dicyclopentyl-1,3-dimethoxypropan
2,2-Dicyclohexyl-1,3-dimethoxypropan.

4. Verfahren zur Herstellung eines Diethers der Formel I, wie in Anspruch 1 definiert, umfassend die Stufe der Umsetzung eines Diols der Formel II 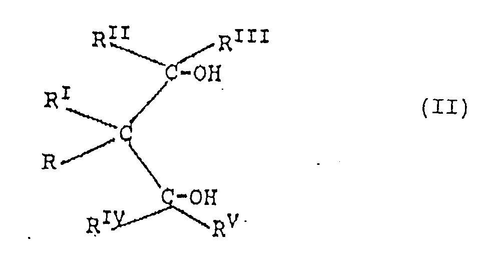 mit Verbindungen der Formel R^{VI}-X, R^{VII}-X oder deren Mischungen (worin X = Cl, Br, J, C₆H₅-SO₃, p-CH₃C₆H₄-SO₃, CH₃SO₃), worin R und R^{I}, R^{II}, R^{III}, R^{IV} und R^{V} die in Anspruch 1 angegebene Bedeutung besitzen.

5. Verfahren gemäß Anspruch 4, worin R^{VI} und R^{VII} für Alkylreste mit 1 bis 6 Kohlenstoffatomen stehen und R^{II}, R^{III}, R^{IV} und R^{V} Wasserstoff bedeuten und R und R^{I} Aryl-, Alkaryl- oder Aralkylreste gemäß Anspruch 1 darstellen.

6. Verfahren gemäß Anspruch 5 zur Herstellung eines Diethers, ausgewählt unter
2,2-Diphenyl-1,3-dimethoxypropan
2,2-Dibenzyl-1,3-dimethoxypropan
2,2-Bis-(cyclohexylmethyl)-1,3-dimethoxypropan
2-Isopropyl-2-cyclohexylmethyl-1,3-dimethoxypropan
2-Isopropyl-2-cyclohexyl-1,3-dimethoxypropan
2-Isopropyl-2-cyclopentyl-1,3-dimethoxypropan
2,2-Dicyclopentyl-1,3-dimethoxypropan
2,2-Dicyclohexyl-1,3-dimethoxypropan.

## Patentansprüche (Patentansprüche für folgende(n) Vertragsstaat(en): ES)

1. Verfahren zur Herstellung eines Diethers der Formel I 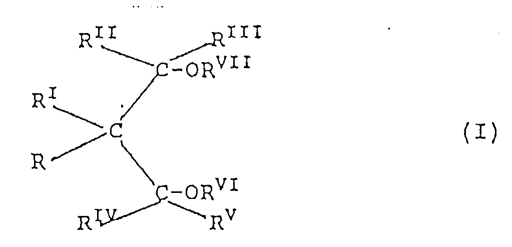 worin R und R^{I}, R^{II}, R^{III}, R^{IV} und R^{V}, gleich oder verschieden, für H oder lineare oder verzweigte Alkyl-, Cycloalkyl-, Aryl-, Alkaryl- oder Aralkylreste mit 1 bis 18 Kohlenstoffatomen stehen, mit der Maßgabe, daß R und R^{I} nicht beide für H stehen, und wenn R und R^{I} Alkylreste sind, diese drei oder mehr Kohlenstoffatome aufweisen; R^{VI} und R^{VII}, gleich oder verschieden, für lineare oder verzweigte Alkyl-, Cycloalkyl-, Aryl-, Alkarylreste mit 1 bis 18 Kohlenstoffatomen stehen; und zwei oder mehrere von R bis R^{VII} unter Bildung einer cyclischen Struktur gebunden sein können; mit der Maßgabe, daß Diether ausgeschlossen sind, worin sämtliche von R^{II}, R^{III}, R^{IV} und R^{V} für H stehen; und sämtliche von R, R^{I}, R^{VI} und R^{VII} Alkylreste, wie vorstehend definiert, sind;
umfassend die Stufe der Umsetzung eines Diols der Formel II 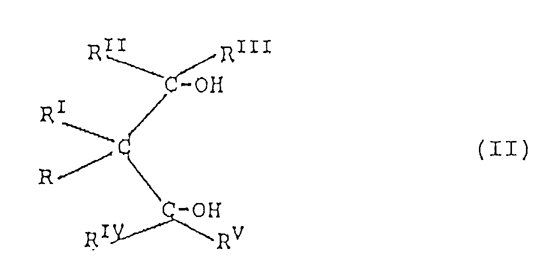 mit Verbindungen der Formel R^{VI}-X, R^{VII}-X oder von deren Mischungen (worin X = Cl, Br, J, C₆H₅-SO₃, p-CH₃C₆H₄-SO₃, CH₃SO₃), worin R und R^{I}, R^{II}, R^{III}, R^{IV} und R^{V} die vorstehend angegebene Bedeutung besitzen.

2. Verfahren gemäß Anspruch 1, worin R^{VI} und R^{VII} Alkylreste mit 1 bis 6 Kohlenstoffatomen bedeuten und R^{II}, R^{III}, R^{IV} und R^{V} Wasserstoff sind und R und R^{I} für Aryl-, Alkaryl- oder Aralkylreste gemäß Anspruch 1 stehen.

3. Verfahren gemäß Anspruch 2 zur Herstellung eines Diethers, ausgewählt unter
2,2-Diphenyl-1,3-dimethoxypropan
2,2-Dibenzyl-1,3-dimethoxypropan
2,2-Bis-(cyclohexylmethyl)-1,3-dimethoxypropan
2-Isopropyl-2-cyclohexylmethyl-1,3-dimethoxypropan
2-Isopropyl-2-cyclohexyl-1,3-dimethoxypropan
2-Isopropyl-2-cyclopentyl-1,3-dimethoxypropan
2,2-Dicyclopentyl-1,3-dimethoxypropan
2,2-Dicyclohexyl-1,3-dimethoxypropan.

## Revendications (Revendications pour l'(les) Etat(s) contractant(s) suivant(s): BE, DE, FR, GB, IT, NL)

1. Diéthers de formule I: 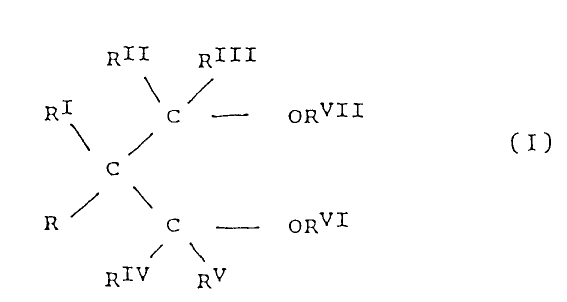 dans laquelle:
R et R^{I}, R^{II}, R^{III}, R^{IV} et R^{V}, identiques ou différents, représentent un atome d'hydrogène ou des radicaux arylalkyle, alkylaryle, aryle, cycloalkyle ou alkyle linéaires ou ramifiés comportant de 1 à 18 atomes de carbone; à condition que lorsque R et R^{I} ne représentent pas tous les deux un atome d'hydrogène et que lorsque R et R^{I} sont des radicaux alkyle, ils comportent au moins 3 atomes de carbone;
R^{VI} et R^{VII}, identiques ou différents, représentent des radicaux alkylaryle, aryle, cycloalkyle ou alkyle, linéaires ou ramifiés, comportant de 1 à 18 atomes de carbone; au moins deux des groupes R à R^{VII} peuvent être liés pour former une structure cyclique; à condition que les diéthers dans lesquels tous les groupes R^{II}, R^{III}, R^{IV} et R^{V} représentent un atome d'hydrogène; et tous les groupes R, R^{I}, R^{VI} et R^{VII} représentent des radicaux alkyle tels que définis ci-dessus, soient exclus.

2. Diéthers selon la revendication 1, dans lesquels R^{VI} et R^{VII} sont des radicaux alkyle comportant 1 à 6 atomes de carbone, R^{II}, R^{III}, R^{IV} et R^{V} sont des atomes d'hydrogène et R et R^{I} représentent des radicaux aryle, alkylaryle ou arylalkyle selon la revendication 1.

3. Diéthers selon la revendication 2, sélectionnés dans le groupe consistant en:
- 2,2-diphényl-1,3-diméthoxypropane;
- 2,2-dibenzyl-1,3-diméthoxypropane;
- 2,2-bis(cyclohexylméthyl)-1,3-diméthoxypropane;
- 2-isopropyl-2-cyclohexylméthyl-1,3-diméthoxypropane;
- 2-isopropyl-2-cyclohexyl-1,3-diméthoxypropane;
- 2-isopropyl-2-cyclopentyl-1,3-diméthoxypropane;
- 2,2-dicyclopentyl-1,3-diméthoxypropane;
- 2,2-dicyclohexyl-1,3-diméthoxypropane.

4. Un procédé de préparation d'un diéther représenté par la formule I, tel que défini dans la revendication 1, comprenant l'étape de réaction:
d'un diol de formule II: 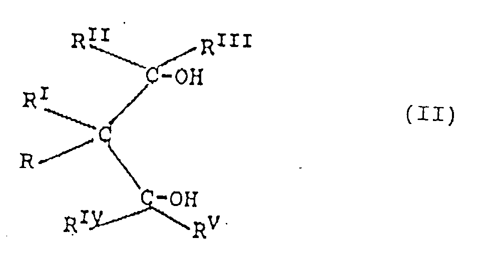 avec des composés représentés par les formules:
R^{VI}-X, R^{VII}-X
ou avec leurs mélanges;
dans lesquelles:
X = Cl, Br, I, C₆H₅-SO₃, p-CH₃C₆H₄-SO₃, CH₃SO₃;
R et R^{I}, R^{II}, R^{III}, R^{IV} et R^{V} sont tels que définis dans la revendication 1.

5. Le procédé selon la revendication 4, dans lequel R^{VI} et R^{VII} sont des radicaux alkyle comportant de 1 à 6 atomes de carbone et R^{II}, R^{III}, R^{IV} et R^{V} sont des atomes d'hydrogène, et R et R^{I} sont des radicaux aryle, alkylaryle ou arylalkyle selon la revendication 1.

6. Le procédé selon la revendication 5 pour la préparation d'un diéther sélectionné dans le groupe consistant en:
- 2,2-diphényl-1,3-diméthoxypropane;
- 2,2-dibenzyl-1,3-diméthoxypropane;
- 2,2-bis(cyclohexylméthyl)-1,3-diméthoxypropane;
- 2-isopropyl-2-cyclohexylméthyl-1,3-diméthoxypropane;
- 2-isopropyl-2-cyclohexyl-1,3-diméthoxypropane;
- 2-isopropyl-2-cyclopentyl-1,3-diméthoxypropane;
- 2,2-dicyclopentyl-1,3-diméthoxypropane;
- 2,2-dicyclohexyl-1,3-diméthoxypropane.

## Revendications (Revendications pour l'(les) Etat(s) contractant(s) suivant(s): ES)

1. Un procédé de préparation d'un diéther de formule I: 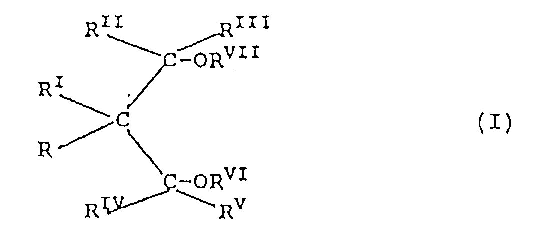 dans laquelle:
R et R^{I}, R^{II}, R^{III}, R^{IV} et R^{V}, identiques ou différents, représentent un atome d'hydrogène ou des radicaux arylalkyle, alkylaryle, aryle, cycloalkyle ou alkyle linéaires ou ramifiés comportant de 1 à 18 atomes de carbone; à condition que R et R^{I} ne représentent pas tous les deux un atome d'hydrogène et que lorsque R et R^{I} sont des radicaux alkyle, ils comportent au moins 3 atomes de carbone;
R^{VI} et R^{VII}, identiques ou différents, représentent des radicaux alkylaryle, aryle, cycloalkyle ou alkyle, linéaires ou ramifiés, comportant de 1 à 18 atomes de carbone; au moins deux des groupes R à R^{VII} peuvent être liés pour former une structure cyclique; à condition que les diéthers dans lesquels tous les groupes R^{II}, R^{III}, R^{IV} et R^{V} représentent un atome d'hydrogène; et tous les groupes R, R^{I}, R^{VI} et R^{VII} représentent des radicaux alkyle tels que définis ci-dessus, soient exclus; comprenant l'étape de réaction:
d'un diol de formule II: 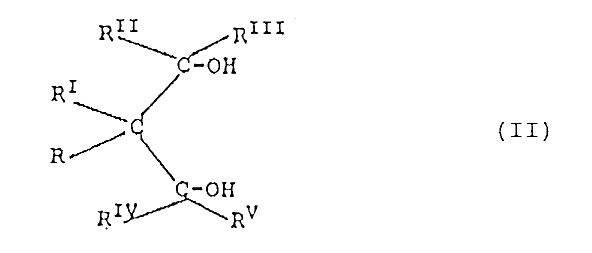 avec des composés représentés par les formules:
R^{VI}-X, R^{VII}-X
ou avec leurs mélanges;
dans lesquelles:
X = Cl, Br, I, C₆H₅-SO₃, p-CH₃C₆H₄-SO₃, CH₃SO₃;
R et R^{I}, R^{II}, R^{III}, R^{IV} et R^{V} sont tels que définis ci-dessus.

2. Le procédé selon la revendication 1, dans lequel R^{VI} et R^{VII} sont des radicaux alkyle comportant de 1 à 6 atomes de carbone et R^{II}, R^{III}, R^{IV} et R^{V} sont des atomes d'hydrogène, et R et R^{I} sont des radicaux aryle, alkylaryle ou arylalkyle selon la revendication 1.

3. Le procédé selon la revendication 2 pour la préparation d'un diéther sélectionné dans le groupe consistant en:
- 2,2-diphényl-1,3-diméthoxypropane;
- 2,2-dibenzyl-1,3-diméthoxypropane;
- 2,2-bis(cyclohexylméthyl)-1,3-diméthoxypropane:
- 2-isopropyl-2-cyclohexylméthyl-1,3-diméthoxypropane;
- 2-isopropyl-2-cyclohexyl-1,3-diméthoxypropane;
- 2-isopropyl-2-cyclopentyl-1,3-diméthoxypropane;
- 2,2-dicyclopentyl-1,3-diméthoxypropane;
- 2,2-dicyclohexyl-1,3-diméthoxypropane.
